Europäisches Patentamt

European Patent Office

Office européen des brevets

(19)

(11) Publication number: **0 104 356**
**B1**

(12) **EUROPEAN PATENT SPECIFICATION**

(45) Date of publication of patent specification: **14.01.87**

(21) Application number: **83107420.8**

(22) Date of filing: **28.07.83**

(51) Int. Cl.⁴: **C 07 K 3/14,** C 07 K 3/20,
C 07 K 3/22, A 61 K 35/16

(54) Highly purified human antihemophilic factor and method for the preparation thereof.

(30) Priority: **05.08.82 US 405456**

(43) Date of publication of application:
**04.04.84 Bulletin 84/14**

(45) Publication of the grant of the patent:
**14.01.87 Bulletin 87/03**

(84) Designated Contracting States:
**DE FR GB IT SE**

(56) References cited:
EP-A-0 041 173
US-A-4 278 594

EXPERIENTA, vo. 35, no. 7, July 1979 B.A.
PERRET et al.: "Separation of factor VIII
multimers under physiological conditions",
page 941
Chemical Abstracts vol. 97, no. 9, 30 August
1982, Columbus, Ohio, USA K. HORIIKE et al.:
"High-speed gel filtration chromatography of
proteins. Evaluation and calibration of a
column of porous silica-based aqueous gels

(73) Proprietor: **THE UNIVERSITY OF ROCHESTER**
**601 Elmwood Avenue**
**Rochester New York 14642 (US)**

(72) Inventor: **Chavin, Stephen I.**
**133 Palmerston Road**
**Rochester New York 14618 (US)**
Inventor: **Fay, Philip J.**
**37 Barker Street**
**Rochester New York 14611 (US)**

(74) Representative: **Wagner, Karl H. et al**
**WAGNER & GEYER Patentanwälte**
**Gewuerzmuehlstrasse 5 Postfach 246**
**D-8000 München 22 (DE)**

(56) References cited:

based on the correlation of distribution
coefficient with Stokes radius", page 300,
column 1, abstract no. 68742b
Chemical Abstracts vol. 94, no. 9, 2 March 1981,
Columbus, Ohio, USA K. G. CHANDY et al.:

Courier Press, Leamington Spa, England.

**0 104 356**

(58) References cited:

"The estimation of Stokes radius for secretory immunoglobulin A, using thin-layer chromatography followed by crossed immunoelectrophoresis", page 542, column 1, abstract no. 63462p
Chemical Abstracts vol. 96, no. 21, 24 May 1982, Columbus, Ohio, USA G. M. ROTHE et al.: "Determination of molecular weights and Stokes' radii of nondenatured proteins by polyacrylamide gradient gel electrophoresis. 1. An equation relating total polymer concentration, the molecular weight of proteins in the range of 104-106, and duration of electrophoresis", page 366, column 1, abstract no. 177334v

## Description

### Field of the invention

This invention relates to and has among its objects the provision of novel methods for the preparation of highly-purified human Antihamophilic Factor preparations. Further objects of the invention will be evident from the following description wherein parts and percentages are by weight unless otherwise specified.

### Description of the prior art

It is known that the clotting of human blood is a complicated process, involving a series of reactions mediated by 13 different factors. It also is well known that a cause of hemophilia is the inability of the afflicted individual to synthesize one of these factors, known variously as antihemophilic factor, AHF, AHG, Factor VIII or Factor VIII C, in amounts sufficient to support adequate clotting. About 40 percent of hemophiliacs have no ability to synthesize AHF, while the others have diminished ability. Dried preparations of AHF concentrate are sold commercially for administration to hemophiliacs for treatment of bleeding or in advance of surgery. The AHF concentrate is obtained from plasma from human donors, through the use of known techniques. At the time of use, the dried concentrate is dissolved in sterile water, and the resulting solution is administered intravenously.

The usual commercial AHF preparation is not pure AHF. Rather, it is an AHF-enriched fraction obtained from plasma and contains other components. It is desirable that the AHF concentrate be as pure as possible, but further improvements in purity through modification of the procedure for isolating AHF from plasma have not been practically feasible due to the difficulty of separating plasma components. AHF is quite difficult to separate and purify because of its low content in the plasma and the instability of its activity. The known AHF concentrates are prepared from fraction I separated from plasma by means of Cohn's ethanol fractionation method or from the cryoprecipitate obtained by freezing a plasma and then thawing it at a low temperature. However, they are all crude products of low purity and contain a large quantity of fibrinogen. If given in large or frequently repeated doses, they may make the state of the patient quite dangerous clinically, bringing an excessively overloaded fibrinogen content in the circulating system. Moreover, it is difficult to determine their accurate dose because of the deviation in the activity in each preparation. For the above-mentioned reasons, the current trend is towards the development of a highly purified, highly concentrated AHF preparation from a large quantity of pooled plasma. The high potency AHF concentrates hitherto disclosed are generally produced by first preparing crude fractions of AHF such as Cohn's fraction I or cryoprecipitate.

The prior art of AHF concentrate fractionation has demonstrated that the concentrate can be separated from fibrinogen and other proteins by column chromatography, polyethylene glycol (PEG) or polypropylene glycol (PPG) precipitation, glycine precipitation (or with other amino acids), as well as alcohol precipitation. British Patent No. 1,507,198 and U.S. Patent No. 3,973,002 utilize pH and temperature adjustments to result in some purification of an AHF concentrate to give low potency preparations of intermediate purity. The processes described in these patents utilize a fraction extracted from the cryoprecipitate rather than cryoprecipitate itself. Furthermore, fractionation stops after one cycle of pH and cooling adjustment. Others have also recognized the effect of using one cycle of pH and cooling adjustment (J. K. Smith *et al, Transfusion* 19, 299—306, [1979]).

The compositions of AHF obtained by the processes discussed above are of relatively low concentration (of the order of about 5 to 15 units of AHF activity per ml) and low purity (less than 1 unit AHF activity per mg protein).

Polyethylene glycol fractionation and glycine-precipitated fractionation to yield AHF concentrates have been described in U.S. Patent Nos, 3,415,804; 3,682,881; 3,770,631; 4,027,013; 3,839,314; 4,069,216; 3,631,018; 3,652,530; 4,073,886; and 4,085,095.

Kisker (*Thromb. Diath. Haemorrhagica*, 17, 381 [1967]), as well as Penick and Brinkhouse (*Amer, J. Med. Sciences*, 232, 434 [1956]), have pointed out in their papers, in the preparative process of such high potency AHF concentrates, particularly in the course of separating and purifying AHF the co-existing prothrombin complex and active forms of its constitutive factors, such as IIa, Xa and the like, are markedly detrimental to the stability of AHF and sometimes irreversibly injure the latter to inactivate it. In order to improve stability, yield and solubility of AHF, therefore, it is quite important and essentially necessary to inactivate or eliminate these instabilization factors at the earliest stage of the separation-purification step. Methods for inactivating said instabilization factors have been disclosed in, for example, U.S. Patent No. 3,803,115. It has also been disclosed that said instabilization factors can be removed by the use of an adsorbent such as aluminium hydroxide, magnesium hydroxide, barium carbonate, barium sulfate, rivanol (6,9-diamino-2-ethoxyacridine lactate), ion-exchange resin (Amberlite® IRC-50), glycine ethyl ester or the like (Bidwell, E. *et al, Brit. J. Haemat.*, 13, 568 [1967]; Soulier, J. P. *et al, Presse med.*, 72, 1223 [1964]; Surgenor, P. M. *et al, J. Phys. Colloid Chem.*, 55, 94 [1951]); Hoag, M. S. *et al, J. Clin. Invest.*, 39, 554 [1960]). Among them, aluminium hydroxide is known to be relatively good adsorbent of said instabilization factors and therefore has been used most frequently (U.S. Patent No. 4,170,639). DEAE-crosslinked dextran has also been used to adsorb and remove the prothrombin complex (U.S. Patent No. 4,093,608).

As mentioned above, the AHF concentrates obtained by the prior art processes discussed above are of relatively low specific activity, namely about one unit or less of AHF activity per milligram (mg) of protein, one of the undesirable impurities being denatured AHF. U.S. Patents 4,289,691 and 4,302,445 disclose processes for preparing AHF preparations having a specific activity of AHF activity of 1—3 units per mg of protein, and U.S. Patent 4,294,826 describes a method for preparing human AHF having a specific activity of about 1—10 units of AHF activity per mg.

B. A. Perlt et al., Experientia, 35 (7), July 1979, page 941 describe a method for fractionating Factor VIII (AHF) into multimers primarily on the basis of electrical charge. The use of a SDS/polyacrylamide-agarose electrophoretic gel as described therein merely serves to retard migration of the multimers in the electric field.

US—A—4 278 594 describes the isolation of a AHF which is free of fibronectin or von Willebrand's factor. There is no indication that the product is free of Factors II, VII, IX or X, or is free of fibrinogen or albumin. The product is not characterized, for example, in terms of specific activity or molecular weight.

Summary of the invention

The invention provides a method for preparing a highly purified, essentially homogenous Antihemophilic Factor concentrate which is, characterized in that it comprises the steps of:—

(a) obtaining an Antihemophilic Factor concentrate which is totally or partially free from prothrombin complex proteins, fibrinogen, and albumin,

(b) subjecting the Antihemophilic Factor concentrate to a separation on the basis of Stoke's radius to separate Antihemophilic Factor of an apparently high Stoke's radius value from other proteins,

(c) treating the Antihemophilic Factor concentrate to change the effective Stoke's radius of the Antihemophilic Factor molecule to an apparently low value,

(d) subjecting the Antihemophilic Factor concentrate to a separation on the basis of Stoke's radius to separate Antihemophilic Factor of apparently low Stoke's radius value from other proteins,

(e) subjecting the Antihemophilic Factor concentrate to chromatography on an anion exchange medium.

The invention further provides an Antihemophilic Factor concentrate having a specific activity of at least about 4000 Antihemophilic Factor units per milligram of protein.

The method of the present invention provides highly purified, biologically active human AHF preparations. In the present method a concentrate containing AHF of intermediate purity, such as a commercial AHF preparation is prepared by fractionation of human blood plasma. The concentrate containing AHF is subjected to a separation on the basis of Stokes' radius to separate AHF from the bulk of proteins in the AHF concentrate. The pooled fractions from above containing AHF activity are concentrated, such as by precipitation, by addition of ammonium sulfate, sodium sulfate, by diafiltration or PEG addition. The concentrate is subsequently solubilized or equilibrated in an aqueous medium and treated to change the effective Stokes' radius of the AHF molecule to an apparently low value and then subjected to a separation on the basis of Stokes' radius to further separate AHF from the concentrate. The separated AHF is treated to remove cations by dialysis and chromatographed on an anion exchange medium to give a highly purified AHF preparation.

The human AHF preparations produced by the method of the invention have about 4000—8000 units of AHF (procoagulant) activity per mg of protein (one unit of activity is that found in 1 ml of normal human plasma). The product having at least about 4000 units of AHF activity per mg of protein appears to be homogeneous and have a molecular weight of 200,000—400,000 daltons by High Performance Liquid Chromatography (HPLC).

The primary advantage of the present invention is that for the first time a human AHF preparation of high purity and high activity is obtainable. As mentioned above the preparations of the invention are homogeneous thus being essentially free of fibrinogen, fibronectin, von Willebrand's protein, Vitamin K-dependent coagulation factors, and other undesirable or destabilizing enzymes or proteins.

The improved process of the invention has the advantage of producing the above human preparation, which has heretofore been unavailable. All molecules of AHF in the present preparation can be cleaved by thrombin, an indication that AHF is in its native (i.e. non-destabilized) state.

Brief description of the drawings

Figures 1—4 are depictions of the chromatographic separations of AHF (Factor VIII C) in the steps of the method of the invention.

Figure 5 is a depiction of the results of the SDS polyacrylamide gel electrophoresis (Laemmli, *Nature, 277*, 680—685, *1970*) in a 6% gel on preparations produced in accordance with the invention.

Description of the preferred embodiments

The starting material of the present invention is a concentrate of AHF obtained from human blood plasma, such as a commercially available AHF preparation. According to standard procedure, frozen (at −20°C or less) human blood plasma is obtained from a plasma collection center. The frozen plasma is treated to give a concentrate of AHF; for instance frozen plasma may be held at a temperature sufficient to produce a cryoprecipitate or treated with ethanol to give Cohn Fraction I. The AHF concentrate is separated from the pooled plasma by conventional techniques, such as centrifugation for cryoprecipitate.

The concentrate of AHF is next treated by known techniques to totally or partially remove other clotting proteins, such as prothrombin complex proteins, fibrinogen, albumin, and other proteins and to further concentrate the AHF. Removal of the above proteins may be accomplished according to known methods such as those described in the aforementioned patents. For example, the above proteins can be removed from a cryoprecipitate concentrate containing AHF by dissolving the cryoprecipitate in an aqueous medium, adding aluminium hydroxide to precipitate unwanted proteins, treating to adjust pH and salt concentration, and ultrafiltering to yield a concentrate of AHF with substantial reduction of the prothrombin complex proteins, fibrinogen and albumin. Alternately or in addition, the above proteins may be removed from the cryoprecipitate concentrate of AHF by polyethylene glycol and/or glycine precipitation.

The so-prepared concentrate of AHF may be treated to reduce its content of water, either partially or totally, by ultrafiltration, lyophilization, or a combination of both.

The concentrate of AHF is subjected to a separation on the basis of Stokes' radius wherein proteins of lower molecular weight are separated from AHF, which exhibits an apparently high Stokes' radius. The above separation may be accomplished in a variety of ways such as subjecting and AHF concentrate to gel permeation chromatography on cross-linked agarose (such as Biogel® A-15 m or Sepharose® CL-4B) or cross-linked polyacrylamide or to a controlled pore size glass bead treatment or to sucrose density gradient ultrafiltration. All of the above techniques are well-known in the art. Preferably, the AHF concentrate is subjected to gel permeation chromatography on; for example, cross-linked agarose or polyacrylamide. After equilibration on the chromatographic medium, AHF is eluted with a buffered salt solution having an ionic strength of about 0.1—0.4 and a pH of about 6.0—7.5.

The fractions containing the eluted AHF from above are pooled and the pool is concentrated by techniques known in the art such as precipitation with ammonium sulfate, sodium sulfate, by diafiltration or by PEG addition. For example, the AHF pool may be treated with ammonium sulfate (30—40%, weight/volume) to precipitate AHF. The AHF, after concentration, is dissolved or equilibrated in an aqueous salt buffer of pH about 6.0—7.5 and ionic strength about 0.1—0.4. If a salt such as ammonium sulfate was added in the course of concentration of the AHF pool, such salt is removed prior to the next step by known techniques such as dialysis or diafiltration.

Next, the AHF concentrate is treated to change the effective Stokes' radius of the AHF molecule to an apparently low value. To this end one may add a source of divalent cations such as calcium or magnesium wherein about 5—10 parts by volume of protein solution with 1 part of a solution about 1—3 M in divalent cation is employed per part by volume of AHF concentrate. The above mentioned reduction in Stokes' radius can also be accomplished by attaining a high ionic strength in solutions of AHF (e.g. about 1—4 M with sodium chloride) or the incubation of AHF with about 0.01—0.001 parts of thrombin per part of AHF concentrate (in units) (Leon W. Hoyer, *Hemophilia and Hemostasis*, "The Factor VIII Complex:Structure and Function", Alan R. Liss, Inc., pg. 7, [1981]).

Following reduction in Stokes' radius of the AHF molecule, the AHF concentrate is treated to remove divalent cations by known procedures such as dialysis or diafiltration and then is subjected to a separation on the basis of Stokes' radius by any of the means described above. Preferably, the AHF concentrate is subjected to gel permeation chromatography on cross-linked agarose or polyacrylamide, chromatographic medium equilibrated and eluted with a buffered salt solution having an ionic strength of about 0.1—0.4 and a pH of about 6.0—7.5.

The eluted fractions containing AHF activity are pooled and optionally concentrated by reduction of water content. To this end the pooled fractions may be concentrated by techniques known in the art such as dialysis or diafiltration. The fractions may be immersed in, for example, solid PEG 20,000 in order to extract water from the solution within a dialysis container. The concentrated material is treated to remove divalent cations by dialysis or diafiltration, against a buffer at a pH of about 6.0—7.5.

Following the above concentration steps, the fraction containing AHF activity is subjected to chromatography on an anion exchange medium such as quaternary aminoethyl (QAE) cellulose, diethylaminoethyl (DEAE) cellulose, or similar anion exchanger.

The chromatographic medium is washed with a buffered aqueous solution having an ionic strength sufficient to remove unbound protein but not AHF, i.e., 0—0.2 (0—0.2 M sodium chloride). Finally the chromatographic medium is eluted with an aqueous solution having an ionic strength sufficient to elute AHF, i.e., about 0.2—0.6 (e.g. 0.2—0.6 M sodium chloride) to give fractions containing AHF, which are then pooled.

The pooled fractions from the above chromatography contain AHF having a specific activity of at least about 4000 AHF units per mg of protein and represent a substantially homogeneous preparation on ion exchange chromatography on QAE cellulose and sodium dodecyl sulfate (SDS) polyacrylamide gel electrophoresis (Laemmli). Such a product has heretofore not been described, disclosed, or suggested by the prior art. Consequently, the pooled fractions constitute a concentrated AHF preparation purified at least about 350,000-fold over that found in plasma, it may be sterile filtered and lyophilized.

The pooled fractions from above may optionally be subjected to HPLC under non-denaturing conditions. For this purpose conventional HPLC apparatus may be employed and the elution of AHF accomplished in a standard manner based on Stokes' radius.

The fractions containing AHF are pooled and represent a 10% yield of product; the AHF has a specific activity of at least about 4,000 AHF units per mg of protein. The fraction may be sterile filtered and treated

5

to reduce its water content either by ultrafiltration, lyophilization or combinations thereof. The AHF preparation exhibits homogeneity by HPLC and SDS polyacrylamide gel electrophoresis (PAGE) (Laemmli). Thus, the AHF preparation is essentially homogeneous and essentially free (i.e., contains less than about 1% combined of non-AHF proteins) of Factors II, VII, IX, X, fibrinogen, albumin, fibronectin, von Willebrand factor, and has essentially no activity in a non-activated partial thromboplastin time assay indicating that the purified protein essentially does not contain activated clotting factors circumventing the AHF dependent step in the clotting cascade.

The AHF preparations of the invention, in addition to having the ability to correct the clotting defect in hemophilic plasma, also exhibit the following characteristics:

(a) The biological activity is increased, and the putative polypeptide chain is altered, following treatment with thrombin. The purified protein can be activated by treatment of thrombin to give 4—20 fold increase in AHF activity.

(b) The biological activity can be blocked by the inhibitors against AHF which are found in certain patients with classical hemophilia. When mixed with a several fold excess (unit:unit) of AHF inhibitor, between 95 and 99% of the measurable AHF activity can be abolished.

(c) The purified native AHF has an elution volume corresponding to a molecular weight of 200—400,000 daltons. After reduction and SDS PAGE, there is a single polypeptide chain with an apparent molecular weight of about 100,000. Following incubation with thrombin, virtually all of the 100,000 polypeptide disappears and is replaced by a single broad band with a molecular weight of 75,000. No additional bands with lower molecular weights are seen.

(d) The purified AHF appears to be substantially free from significant protease activity, and from essentially all other plasma proteins.

(e) The purified AHF is substantially free from AHF antigen (Factor VIII:C Ag). The ratio of AHF:AHF antigen is usually about 50:1 or greater, usually within the range of about 50:1—150:1.

The amino acid composition of the human AHF preparation of the invention is given in Table 1.

TABLE 1
Human AHF amino acid composition

| | | |
|---|---|---|
| Asp | = | 9.6% |
| Thr | = | 3.65% |
| Ser | = | 5.49% |
| Glu | = | 13.55% |
| Gly | = | 10.5% |
| Ala | = | 7.73% |
| Val | = | 3.75% |
| Met | = | 2.44% |
| Ile | = | 2.67% |
| Leu | = | 11.65% |
| Tyr | = | 4.78% |
| Phe | = | 5.32% |
| Lys | = | 4.29% |
| His | = | 3.01% |
| Arg | = | 8.90% |
| Cysteic Acid | = | 2.62% |

(percentages are weight/weight)

Example

The invention is further demonstrated by the following illustrative examples.

Assays

1) Clotting Assays. AHF was measured by a one-stage clotting assay, using as substrate plasma from a patient with severe AHF deficiency (less than 1% AHF), and using a Fibrometer to determine the clotting time. One unit of AHF was defined as the amount in 1 ml of pooled normal human plasma. (Langdell et al, J. Lab. Clin. Med., 41, 637—644, 1953.

2) Protein estimations were made using a Gilford 250 spectrophotometer. All samples were read at 280 nm and 320 nm, and the absorbance corrected for light scattering by the following formula.

$$A_{280\ corrected} = A_{280\ uncorrected} - 1.7\ (A_{320})$$

A mean $A_1^{1\%}$ cm of 10.0 was assumed for calculations of specific activity.

3) Polyacrylamide gel electrophoresis. Sodium dodecyl sulfate-polyacrylamide gel electrophoresis was performed according to O'Farrell employing a 4.5% stacking gel with a 6% separating gel. Electrophoresis was at 25 mA for four hours. Polyacrylamide gel electrophoresis under nondenaturing conditions employed a 5% gel and 25 mM Tris and 192 mM glycine, pH 8.3. Electrophoresis was at 250 volts for three hours at 4°C (O'Farrell, J. Biol, Chem., 250, 4007—4012 1975). Gels were stained for protein using silver nitrate. Protein was eluted from the unstained gel by slicing at 5 mm intervals and adding each slice to 0.3

6

ml of 50 mM Tris-hydrochloride, pH 7.0, 150 mM NaCl and 200 microgram/ml ovalbumin. The mixture was incubated at 4°C for 2—3 hours and assayed for AHF procoagulant activity as described above.

4) Factor VIII:C Ag assay. The procedure of Reisner et al was followed. The antibody was kindly provided by Howard Reisner. (Reisner et al, Thromb, Research, 14, 135—239, 1979).

5) Inhibition of AHF activity. This was measured by mixing 1 volume of sample containing AHF with an equal volume of human plasma containing inhibitor to AHF. The concentration of inhibitor was always several fold in excess of the AHF concentration. After incubation for the time indicated, the residual AHF was assayed.

6) Thrombin activation of AHF. (Switzer et al, J. Biol. Chem., 255, 10606—10611, 1980).

7) Von Willebrand protein assay. (Voller et al, Bull. World Health Organ., 53, 55—63, 1976).

8) Silver stain. (Merrill et al, Science, 211, 1437—1438, 1980).

Example
Purification of AHF
The starting material was AHF therapeutic concentrate (Koāte®) from Cutter Laboratories, Inc.

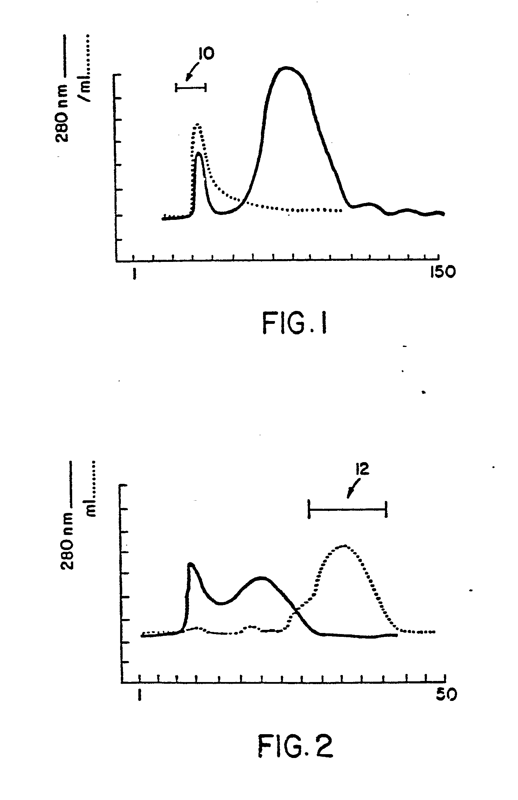

1) Referring to Figure 1 gel permeation chromatography was carried out on Biogel® A-15 m [Bio Rad Laboratories, 150 μm—75 μm (100—200 mesh)] in a column 2.6×90 cm with CaCl$_2$ (1 mM), sodium citrate (5 mM), 0.135 M NaCl, 5% dextrose, 0.1% sodium azide at pH 7.35 and at ambient temperature as the eluant. The fractions containing the highest concentrations of AHF were pooled (10) and concentrated by precipitation with 40% w/v ammonium sulfate. In preparation for the next step, the precipitate was dissolved in 50 mM imidazole buffer at pH 7.0 containing 150 mM NaCl and dialyzed to remove residual ammonium sulfate.

2) The dialyzed sample from 1 above was made 250 mM in CaCl$_2$ by mixing 1 volume of 2.5 M CaCl$_2$ with 9 volumes of protein solution. The sample was then chromatographed on a 2×100 cm glass column of Sepharose® 4B-CL previously equilibrated with the same buffer. The elution medium was a 50 mM imidazole buffer at pH 7.0 containing 150 mM NaCl and 0.25 M CaCl$_2$. Referring to Figure 2, area 12 represents the fractions containing AHF activity, which were pooled.

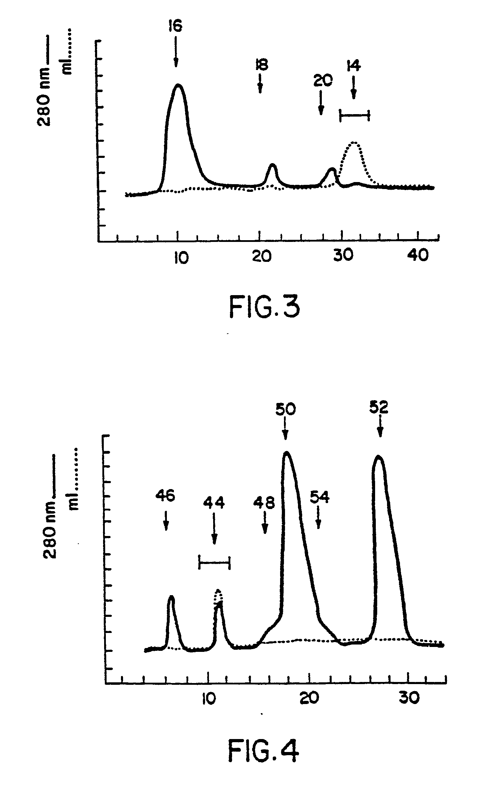

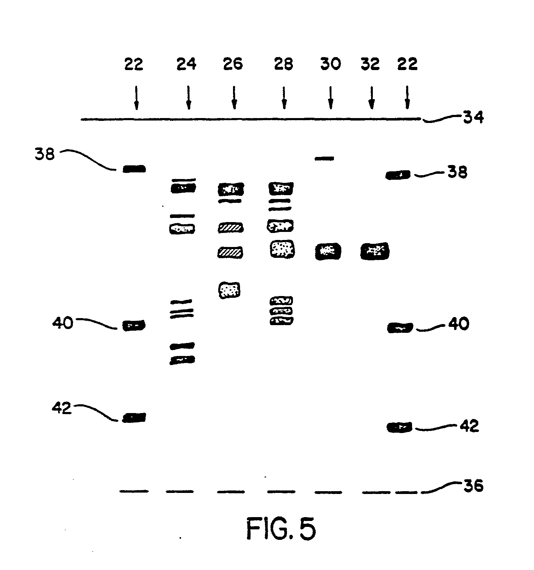

3) The AHF pool (12) from the previous step was concentrated by placing the pool in a dialysis bag and immersing the bag in solid PEG-20,000. The concentrated sample was then dialyzed against 50 mM imidazole buffer pH 7.0 and 0.15 molar sodium chloride and was chromatographed on a 2.5×10 cm plastic column of QAE cellulose previously equilibrated with the same buffer. All of the AHF was bound under these conditions. Referring now to Figure 3, when the unbound protein (16) had been washed through with 0.15 M NaCl, a step gradient of 0.20 M NaCl in the same buffer was run, an additional peak (18) of protein containing little or no AHF was eluted. Finally, a linear gradient of 180 ml from 0.20—1.0 M NaCl was run at 17 ml per hour. An additional peak (20) of protein was eluted, and towards the end of this peak, the AHF activity eluted in a fairly sharp peak (14) starting at around 0.3 M NaCl. The protein was pooled according to the AHF activity. Unbound proteins are indicated at 24 in Figure 5 (SDS polyacrylamide gel electrophoresis [Laemmli] in a 6% gel); protein eluted with 0.2 N NaCl is represented at 26; and peak at 20 of Figure 4 is represented at 28 in Figure 5.

The AHF preparation so-produced exhibited homogeneity (30) on SDS polyacrylamide gel electrophoresis (Laemmli) in a 6% gel (Figure 5). The specific activity of this preparation was about 5,000 units of AHF activity per mg of protein, representing a 350,000 fold purification over source plasma. A 10% overall recovery from the commercial AHF concentrate was realized.

4) The pooled fraction from step 3) was concentrated prior to high performance liquid chromatography (HPLC). HPLC was carried out on a Beckman TSK 4000 I×30 cm column at 2,76 bar (40 psi), flow rate of 0.5 ml/hour. The profile is shown in Figure 4. The AHF eluted coincident with the second peak (44), intermediate between the elution positions of IgM (MW 890,000) and IgG (MW 160,000). The two largest peaks 50 and 52 do not contain AHF activity and have not been characterized. Peak 54 represents bovine serum albumin (BSA). In Figure 4, arrows at 46, 48, and 54 represent elution volumes of marker proteins of known molecular weights in parallel runs: 46=IgM (MW 890,000) and 48=IgG (MW 160,000).

Referring to Figure 5, the AHF pool from step 4 above is essentially homogeneous (32) on SDS PAGE (Laemmli) in a 6% gel. This preparation has a specific activity of about 5000 AHF units per mg of protein representing a purification factor of 350,000 over source plasma. In Figure 5 the origin for the SDS PAGE is represented at 34 and the ion front at 36. Column 22 represents myosin heavy chain (38) molecular weight 200,000, BSA (40) molecular weight 68,000, and ovalbumin (42) molecular weight 43,000.

The above operations and results are summarized in Table 2.

TABLE 2
Purification of human AHF

| | Volume (ml) | Total protein (mg) | Total activity (units) | Specific activity (units/mg) | Yield (%) | Purification (×-fold) |
|---|---|---|---|---|---|---|
| Plasma | — | — | — | 0.014 | — | 1 |
| Koate® | 250 | 12,580 | 11,000 | 0.87 | 100 | 62 |
| Biogel A-15 M ammonium sulfate dialysis | 22 | 318 | 9,469 | 29.8 | 86 | 2,126 |
| Ca⁺⁺dissociation Sepharose CL-4B dialysis | 110 | 13.2 | 6,850 | 519 | 62 | 37,067 |
| QAE cellulose | 29.5 | 0.28 | 1,396 | 4,986 | 12.7 | 356,122 |

All values represent the average of two preparations.

Carbohydrate analysis of AHF according to the invention
1. Periodic acid—Schiff staining
    The highly purified AHF was subjected to SDS-polyacrylamide gel electrophoresis using a 10% separating gel. Following electrophoresis, the gel was stained for carbohydrate using the periodic acid—Schiff's (PAS) stain (Dubray and Bezard, 1982). AHF is stainable using this procedure indicating that it is a glycoprotein (Fig. 6, lane 2).

2. Effects of glycosidases on electrophoretic mobility of AHF
    AHF was treated with different combinations of exoglycosidases for 18—24 h at 40 prior to electrophoresis using an 8% SDS-polyacrylamide gel. Following electrophoresis, the gel was stained with silver nitrate. The electrophoretic mobility of AHF was increased following treatment with neuraminidase (0.1 unit/ml). The addition of three other exoglycosidases, including β-gelactosidase (1.5 unit/ml), α-mannosidase (5 μg/ml) or N-acetylglucosaminidase (5 μg/ml) to neuraminidase-treated AHF resulted in no further change in electrophoretic mobility. The addition of all four exoglycosidases to AHF showed no change over that observed with neuraminidase alone. A comparison run with antithrombin III in the absence and presence of all four exoglycosidases respectively, showed the utility of gel electrophoresis to qualitate changes in electrophoretic mobility resulting from the removal of carbohydrate residues from glycoproteins. A test wih BSA in the absence and presence of the four glycosidases, respectively, showed that no proteolytic activity was present in the glycosidase mixture.
    Treatment of AHF with the endoglycosidases D and H either individually, in combination with each other, or in combination with the exoglycosidases showed no additional effects on electrophoretic mobility.
    When endo- and exo-glycosidase-treated AHF was subjected to electrophoresis followed by PAS staining, an increase in electrophoretic mobility and a decrease in staining intensity was observed (Fig. 6, lane 3) relative to the untreated AHF (Fig. 6, lane 2). A similar result was obtained using antithrombin III in the absence (Fig. 6, lane 4) and presence (Fig. 6, lane 5) of the endo- plus exoglycosidases.
    AHF (Mr 100,000) is cleaved by thrombin into two fragments of Mr 75,000 and 25,000. [125]-I-labelled AHF was treeated with the exoglycosidase cocktail following thrombin cleavage of AHF. The reaction mixtures were subjected to 10% SDS-polyacrylamide gel electrophoresis followed by autoradiography. It was found that AHF treated with thrombin yields, two fragments (Mr 75,000 and ~25,000 polypeptides). After thrombin treatments, the AHF was treated with the exoglycosidase cocktail. The Mr 75,000 polypeptide was not significantly changed in electrophoretic mobility while the Mr ~25,000 polypeptide showed a significant increase in electrophoretic mobility suggesting that this polypeptide is relatively rich in carbohydrate (at least sialic acid) relative to the Mr 75,000 polypeptide.

3. Effects of Glycosidases on AHF coagulant activity
    AHF treated with individual exo- and endo-glycosidases or in combination was assayed for clotting activity. While some veriability was observed, for the most part no significant increase or decrease in clotting activity was observed relative to untreated AHF.

In vivo survival of glycosidase treated AHF
    Purified AHF was trace labelled with $^{125}$I using lactoperoxidase and glucoseòxidase coupled to

8

**0 104 356**

insoluble beads. The labelled protein was separated from non-covalently bound iodine using gel filtration followed by dialysis. AHF labelled by this procedure retains 10—20% of its clotting activity and migrates in a reduced SDS PAGE in a single polypeptide chain $M_r$ 100,000 with the same electrophoretic properties as its unlabelled counterpart.

Approximately 50—100 ng of labelled AHF ($13 \times 10^6$ cpm) were injected carefully into the central ear vein of a 2 kg New Zealand white rabbit. Samples of blood were withdrawn after different time intervals, from an indwelling scalp vein needle in the opposite ear. Samples of 1 ml were removed, mixed with 1/100 volume of 40% citrate, and counted in a gamma counter. The amount of labelled protein remaining was plotted against time, in order to determine the rate of clearange of labelled protein.

The results of 3 separate experiments in 2 animals indicate that about 50% of the labelled protein disappears with a half time of ~60 minutes, whereas most of the remainder continues to circulate with a half time of about 4 hours. A small fraction of the AHF, perhaps ~20%, appears to have an even longer half life, in the range of 10—12 hours.

Several samples of plasma from different times after infusion were analyzed by SDS PAGE. The results confirm that the radioactivity continues to be associated covalently with the protein.

These results indicate that purified human AHF, in the absence of detectable amounts of other proteins, circulates in rabbits with a biological half time of at least several hours, and that at the end of 4—8 hours, a significant amount of the protein remains in the plasma. (It is not yet known how this behavior compares with that of AHF in the form of Koate® or cryoprecipitate, when the latter are given to rabbits).

A similar infusion experiment was carried out with AHF which was treated for 15 hours with neuraminidase to remove terminal sialic acids. This treatment has been shown to have no effect on the clotting activity of AHF in vitro, and to cause a small increase in the rate of migration of AHF in SDS PAGE.

When the desialylated AHF was infused intravenously, about 75% of the activity disappeared from the plasma within 5 minutes of infusion. The remaining 25% remained in circulation with a biological half time of ~3—4 hours. The results with the desialylated AHF are similar to those seen with many other (but not all) plasma glycoproteins. Removal of the terminal sialic acids results in the very rapid clearance from the plasma of a major fraction of the protein. This effect on clearance time is in contrast to the lack of detectable effect on clotting activity in vitro, as reported earlier.

Listed below is the information concerning the glycosidase experiments performed with AHF:

Glycosidases:
 Exoglycosidases
  neuraminidase from *cl. perfringens* Type VIII Product No. N-5631 Sigma
  β-galactosidase from *E. coli* EC 3.2.1.23 No. 105 031 Boehringer Mannheim
  α-mannosidase from Jack bean EC 3.2.1.24 No. 107 379 Boehringer Mannheim
  β-N-Acetyl-D-glucosaminidase from beef kidney EC 3.2.1.30 No. 101 915 Boehringer Mannheim

 Endoglycosidases
  Endoglycosidase D (Endo-β-N-acetylglucosaminidase D) from *Diplococcus pneumonial*
  Endoglycosidase H (Endo-β-N-acetylglucosaminidase H) from *Streptomyces plicatus*

Iodination of AHF
  $^{125}I^+$ labelling of AHF and the separation of $^{125}I$-AHF from free iodine$^{125}$ used the methods described in the Bio-Rad technical bulletin #1071 on Enzymobeads.

Densitometer scans of PAS stained gels of AHF were performed using a Quick Scan Jr. and Quick Quant II-T from Helena Laboratories.

TABLE 3
Clotting activity of glycosidase-treated AHF

| Conditions | Activity (%±Std. deviation) | No. of determinations |
|---|---|---|
| Control (Untreated AHF) | 100% | 3 |
| Exoglycosidase treated AHF | 127±23 | 3 |
| Exo- and Endoglycosidase treated AHF | 95±21 | 3 |

Reaction conditions
All reactions contained purified AHF (~200 units/ml) in a solution containing 20 mM imidasole HCl pH 7.0 150 mM NaCl, 10 mM $CaCl_2$, 100 mM lysine-1 and 0.02% NaAzide. The reaction volume was 100 ml.

9

Reactions involving exoglycosidases contained neuraminidase (0.1 unit/ml) β-galactosidase (0.1 unit/ml), α-mannosidase (0.05 units/ml) and N-acetyl glucosamidase (0.1 unit/ml).

Reactions with exo- and endoglycosidases contained the above enzymes plus endoglycosidase D (0.02 units/ml) and endoglycosidase H (0.02 units/ml).

All reactions were incubated for 18—20 hours at 37°C.

At the end of the reaction, an aliquot was removed to determine the clotting activity (these values are reported in Table 3). The remainder of the reaction mixture was electrophoresed in an 8% denaturing, polyacrylamide gel. After electrophoresis, the gel was stained for carbohydrate using periodic acid— Schiffs reagent (PAS). This staining procedure allows for the determination of relative amounts of carbohydrate in the protein by comparing the intensities of the stain, as determined by scanning densitometry.

It was found that very little, if any sugar is removed by the exoglycosidase mixture alone, but that about 50% of the total carbohydrate is removed from AHF when treated with the combination of exo- plus endoglycosidases.

In vivo survival of purified AHF

AHF was labelled with $^{125}$Iodine. The radiolabelled protein was separated from unreacted $^{125}$I and injected into a rabbit. The 1/2 disappearance time was about 4 hours.

## Claims

1. A method for preparing a highly purified, essentially homogeneous Antihemophilic Factor concentrate, characterized in that it comprises the steps of:—

(a) obtaining an Antihemophilic Factor concentrate which is totally or partially free from prothrombin complex proteins, fibrinogen, and albumin,

(b) subjecting the Antihemophilic Factor concentrate to a separation on the basis of Stoke's radius to separate Antihemophilic Factor of an apparently high Stoke's radius value from other proteins,

(c) treating the Antihemophilic Factor concentrate to change the effective Stoke's radius of the Antihemophilic Factor molecule to an apparently low value,

(d) subjecting the Antihemophilic Factor concentrate to a separation on the basis of Stoke's radius to separate Antihemophilic Factor of apparently low Stoke's radius value from other proteins,

(e) subjecting the Antihemophilic Factor concentrate to chromatography on an anion exchange medium.

2. A method according to claim 1, characterized in that the separation in step (b) or step (d) is accomplished by subjecting the Antihemophilic Factor concentrate to gel permeation chromatography on cross-linked agarose or cross-linked polyacrylamide.

3. A method according to claim 1, characterized in that the separation in Step (b) and/or step (d) is accomplished by subjecting the Antihemophilic Factor concentrate to controlled pore glass bead chromatography.

4. A method according to claim 1, characterized in that the separation in step (b) and/or step (d) is accomplished by sucrose density gradient ultrafiltration.

5. A method according to any of claims 1 to 4, characterized in that the Antihemophilic Factor concentrate in step (c) is treated with a source of divalent cations in an amount of about 5—10 parts by volume of Antihemophilic concentrate per part of a solution about 1—3 M in divalent cations.

6. A highly purified, essentially homogenous Antihemophilic Factor concentrate obtainable by the method of any of claims 1 to 5.

7. An Antihemophilic Factor concentrate having a specific activity of at least about 4000 Antihemophilic Factor units per milligram of protein.

8. An Antihemophilic Factor concentrate according to claim 7, characterized in that it is essentially free from Factors II, VII, IX, and X, fibrinogen, albumin, fibronectin, and von Willebrand factor.

9. An Antihemophilic Factor concentrate according to any of claims 6 to 8, characterized in that it has the amino acid composition of the Antihemophilic Factor as given in Table 1.

10. An Antihemophilic Factor concentrate according to any of claims 6 to 9, characterized in that the ratio of Antihemophilic Factor to Antihemophilic Factor antigen is about 50:1 or greater.

## Patentansprüche

1. Verfahren zur Herstellung eines hochgereinigten im wesentlichen homogenen antihämophilen Faktorkonzentrats dadurch gekennzeichnet, daß es die folgenden Schritte aufweist:

(a) Erhalt eines antihämophilen Faktorkonzentrats, welches vollständig oder teilweise von Prothrombinkomplexproteinen, Fibrinogen und Albumin frei ist,

(b) Aussetzen des antihämophilen Faktorkonzentrats gegenüber einer Trennung auf der Basis des Stoke'schen Radius zur Trennung des antihämophilen Faktors mit einem scheinbar hohen Stoke-Radius-Wert von anderen Proteinen,

**0 104 356**

(c) Behandlung des antihämophilen Faktorkonzentrats zur Änderung des effektiven Stoke'schen Radius des anthämophilen Faktormoleküls auf einen scheinbar niedrigen Wert,

(d) Aussetzen des antihämophilen Faktorkonzentrats gegenüber einer Trennung auf der Basis des Stoke'schen Radius zur Trennung der antihämophilen Faktors mit scheinbar niedrigem Stoke'schen Radius-Wert von anderen Proteinen,

(e) Aussetzen des antihämophilen Faktorkonzentrats gegenüber Chromatographie auf einem Anionenaustauschmedium.

2. Verfahren nach Anspruch 1 dadurch gekennzeichnet, daß die Trennung gemäß Schritt b oder Schritt d dadurch erreicht wird, daß man das antihämophile Faktorkonzentrat der Gelpermeationschromatographie auf vernetzter Agarose oder vernetztem Polyacrylamid aussetzt.

3. Verfahren nach Anspruch 1 dadurch gekennzeichnet, daß die Trennung gemäß Schritt b und/oder Schritt d dadurch erreicht wird, daß man das antihämophile Faktorkonzentrat der gesteuerten Porenglaskugelchromatographie aussetzt.

4. Verfahren nach Anspruch 1 dadurch gekennzeichnet, daß die Trennung in Schritt b und/oder Schritt d durch Sucrosedichtegradientenultrafiltration erreicht wird.

5. Verfahren nach einem der Ansprüche 1 bis 4 dadurch gekennzeichnet, daß das antihämophile Faktorkonzentrat in Schritt c behandelt wird mit einer Quelle von zweiwertigen Kationen in einer Menge von ungefähr 5 bis 10 Volumenteilen antihämophilen Konzentrats pro Teil einer Lösung mit ungefähr 1 bis 3 M in zweiwertigen Kationen.

6. Hochgereinigtes im wesentlichen homogenes antihämophiles Faktorkonzentrat erhältlich durch das Verfahren nach einem der Ansprüche 1 bis 5.

7. Antihämophiles Faktorkonzentrat mit einer spezifischen Aktivität von mindestens ungefähr 4000 antihämophilen Faktoreinheiten pro Milligramm Protein.

8. Antihämophiles Faktorkonzentrat nach Anspruch 7 dadurch gekennzeichnet, daß es im wesentlichen frei von den Faktoren II, VII, IX, und X, Fibrinogen, Albumin, Fibronectin und von Willebrand-Faktor ist.

9. Antihämophiles Faktorkonzentrat nach einem der Ansprüche 6 bis 8 dadurch gekennzeichnet, daß es die Aminosäurezusammensetzung des antihämophilen Faktors gemäß Tabelle 1 besitzt.

10. Antihämophiles Faktorkonzentrat nach einem der Ansprüche 6 bis 9 dadurch gekennzeichnet, daß das Verhältnis aus antihämophilen Faktor zu antihämophilen Faktorantigen ungefähr 50:1 oder größer ist.

**Revendications**

1. Un procédé pour préparer un concentré essentiellement homogène et hautement purifié de facteur antihémophilique, caractérisé en ce qu'il comprend les stades consistant à:

(a) obtenir un concentré de facteur antihémophilique qui est totalement ou partiellement dépourvu des protéines du complexe prothrombique, du fibrinogène et de l'albumine;

(b) soumettre le concentré de facteur antihémophilique à une séparation en fonction du rayon de Stokes pour séparer le facteur antihémophilique ayant une valeur apparemment élevée du rayon de Stokes d'avec les autres protéines;

(c) traiter le concentré de facteur antihémophilique pour modifier le rayon de Stokes effectif de la molécule de facteur antihémophilique pour qu'il ait une valeur apparemment faible;

(d) soumettre le concentré de facteur antihémophilique à une séparation en fonction du rayon de Stokes pour séparer le facteur antihémophilique ayant une valeur apparemment faible du rayon de Stokes d'avec les autres protéines;

(e) soumettre le concentré de facteur antihémophilique à une chromatographie sur un milieu échangeur d'anions.

2. Un procédé selon la revendication 1, caractérisé en ce que la séparation dans le stade (b) ou le stade (d) est effectuée en soumettant le concentré de facteur antihémophilique à une chromatographie par filtration sur gel sur de l'agarose réticulée ou du polyacrylamide réticulé.

3. Un procédé selon la revendication 1, caractérisé en ce que la séparation dans le stade (b) et/ou le stade (d) est effectuée en soumettant le concentré de facteur antihémophilique à une chromatographie sur des perles de verre de porosité ajustée.

4. Un procédé selon la revendication 1, caractérisé en ce que la séparation dans le stade (b) et/ou le stade (d) est effectuée par ultrafiltration sur un gradient de densités de saccharose.

5. Un procédé selon l'une quelconque des revendications 1 à 4, caractérisé en ce que le concentré de facteur antihémophilique dans le stade (c) est traité avec une source de cations divalents en une quantité d'environ 5 à 10 parties en volume de concentré antihémophilique par partie d'une solution, environ 1—3 M en cations divalents.

6. Un facteur antihémophilique essentiellement homogène et hautement purifié pouvant être obtenu selon le procédé de l'une quelconque des revendications 1 à 5.

7. Un concentré de facteur antihémophilique ayant une activité spécifique d'au moins environ 4 000 unités de facteur antihémophilique par milligramme de protéines.

8. Un concentré de facteur antihémophilique selon la revendication 7, caractérisé en ce qu'il est essentiellement dépourvu des facteurs II, VII, IX et X, de fibrinogène, d'albumine, de fibronectine et du facteur de von Willebrand.

**0 104 356**

9. Un concentré de facteur antihémophilique selon l'une quelconque des revendications 6 à 8, caractérisé en ce qu'il a la composition des aminoacides du facteur antihémophilique comme indiqué dans le Tableau 1.

10. Un concentré de facteur antihémophilique selon l'une quelconque des revendications 6 à 9, caractérisé en ce que le rapport du facteur antihémophilique à l'antigène du facteur antihémophilique est d'environ 50/1 ou plus.

FIG. I

FIG. 2

1

FIG.3

FIG.4

FIG. 5